# EUROPEAN PATENT SPECIFICATION

(11) **EP 3 930 569 B1**
(45) Date of publication and mention of the grant of the patent: **06.05.2026**
(21) Application number: 20706510.3
(22) Date of filing: 25.02.2020
(51) Int. Cl.: A61B 5/03, A61B 5/00

(54) **ESOPHAGEAL PRESSURE MEASUREMENT CATHETER**
KATHETER ZUR MESSUNG DEX SPEISERÖHRENDRUCKS
CATHÉTER DE MESURE DE PRESSION OESOPHAGIENNE

(30) Priority: 28.02.2019 NL 2022650
(43) Date of publication of application: 05.01.2022
(73) Proprietor: PulmoTech B.V., 9725 AC Groningen (NL)
(72) Inventor: FLINK, Rutger Christiaan, 9725 AC GRONINGEN (NL); HOGENBIRK, Laurens Bastiaan Josef, 9725 AC GRONINGEN (NL); SCHAART, Teunis Hendrik, 9725 AC GRONINGEN (NL)
(74) Representative: Hepp Wenger Ryffel AG
(86) International application number: PCT/EP2020/054922
(87) International publication number: WO 2020/173948

(56) References cited:
- US-A1- 2001 053 920
- US-A1- 2010 319 702
- US-A1- 2013 281 885
- US-A1- 2016 074 581
- US-A1- 2017 136 220

## Description

The invention relates to a catheter comprising a tube having a proximal end and a distal end, wherein the tube is provided with at least one pressure sensor for measuring a pressure, and wherein the tube is provided with at least one inflatable balloon adjacent to the at least one pressure sensor.

Such a catheter is disclosed in US 2017/136220 A1, which relates generally to catheter devices for the site-specific delivery of agents to biological spaces such as blood vessels for treatment of said blood vessels and/or other organ systems. **In** other embodiments other target spaces that may be accessed by the distal end of the catheter include the gastroesophageal tract. The catheter is designed with at least two occlusion balloons, one proximal and one distal, which are large enough so as to produce a closed off agent delivery segment. The catheter optionally comprises a pressure sensing means, whereby the pressure of the fluid environment in the closed off agent delivery segment can be measured.

The current invention relates to different type of catheters, namely an esophageal pressure measurement catheter which is specifically designed for measuring pressures in a patient's esophagus.

US 2013/281885 A1 discloses a method for monitoring physiologic conditions of a patient by inserting an esophageal or other suitable tube into the patient. The tube comprising a lumen having a proximal end, a distal end, and central portion, and a pressure sensor disposed about at least a portion of an outer surface of the lumen of the tube. The pressure sensor is capable of detecting a change in pressure when a force is exerted against the outer surface of the lumen. The medical device may be an endotracheal tube, an esophageal feeding tube, a catheter, or a dermal pad.

A problem when measuring pressures in a patient's esophagus is that any interaction between the esophagus wall and the pressure sensor, such as the wall touching the pressure sensor, may influence the measurement in a negative manner. The invention aims at a more reliable esophageal pressure measurement catheter.

To that end, the tube comprises a feeding lumen for transporting liquid food, medication and/or gastric acid to and/or from the stomach of a patient, the balloon is inflatable to a diameter of between 7 mm and 18 mm, and a distance between an edge of the balloon and the centre of the pressure sensor is less than 20 mm. The balloon, when inflated, acts as a spacer to keep the wall of the esophagus away from the pressure sensor, such that the measurement is more accurate.

Said at least one pressure sensor preferably is a solid state pressure sensor. The tube preferably comprises a pressure sensor lumen for accommodating a connection for the at least one pressure sensor. The tube preferably comprises a balloon inflation lumen for transporting a fluid, such as air, to the at least one inflatable balloon.

The balloon, when inflated under normal, nominally designed operating conditions, preferably has a diameter of between 7 mm and 18 mm, more preferably between 10 mm and 15 mm. The distance between an edge of the balloon and the centre of the pressure sensor is preferably less than 10 mm, more preferably 7 mm, even more preferably less than 5mm, most preferably less than 3 mm. Preferably the balloon has a spherical shape, or the balloon has a cylindrical shape with a substantially rectangular longitudinal cross section. Preferably the balloon is located at the side of the pressure sensor that is nearer to the proximal end than to the distal end of the tube. The balloon is preferably made of urethane, PET and/or nylon. The wall thickness of the balloon is preferably between 0.01 mm and 0.06 mm. The balloon may have a leg on either side for fixating the balloon on the tube, and said leg is less preferably than 7 mm, more preferably less than 5 mm, most preferably less than 3 mm.

The length of the tube is preferably at least 80 cm, preferably at least 95 cm. The diameter of the tube is preferably between 2 mm and 6 mm. The tube is preferably provided with two of said inflatable balloons adjacent and at each side of the at least one pressure sensor, wherein a distance between an edge of each of the balloons and the centre of the pressure sensor is less than 20 mm, preferably less than 10 mm, more preferably less than 7 mm, even more preferably less than 5 mm, most preferably less than 3 mm. Said tube is preferably provided with a plurality of said pressure sensors distributed along a length of the tube, each sensor being provided with at least one inflatable balloon adjacent the at least one pressure sensor, wherein a distance between an edge of the balloon and the centre of the pressure sensor is less than 20 mm, preferably less than 10 mm, more preferably less than 7 mm, even more preferably less than 5mm, most preferably less than 3 mm. At least one of said pressure sensors is preferably provided near the distal end of the tube, for instance in the last 25%, 10% or 5% of the length of the tube.

The invention will now be exemplified by means of preferred embodiments, with reference to the drawings, in which:
Figure 1 is a side view of an esophageal pressure measurement catheter in accordance with the invention;
Figure 2 is a top view of the esophageal pressure measurement catheter of figure 1;
Figure 3 is a cross section of the esophageal pressure measurement catheter of figure 1;
Figure 4 is a side view of a balloon for use with the esophageal pressure measurement catheter in accordance with the invention; and
Figure 5 is a side view of another embodiment of a balloon for use with the esophageal pressure measurement catheter in accordance with the invention.

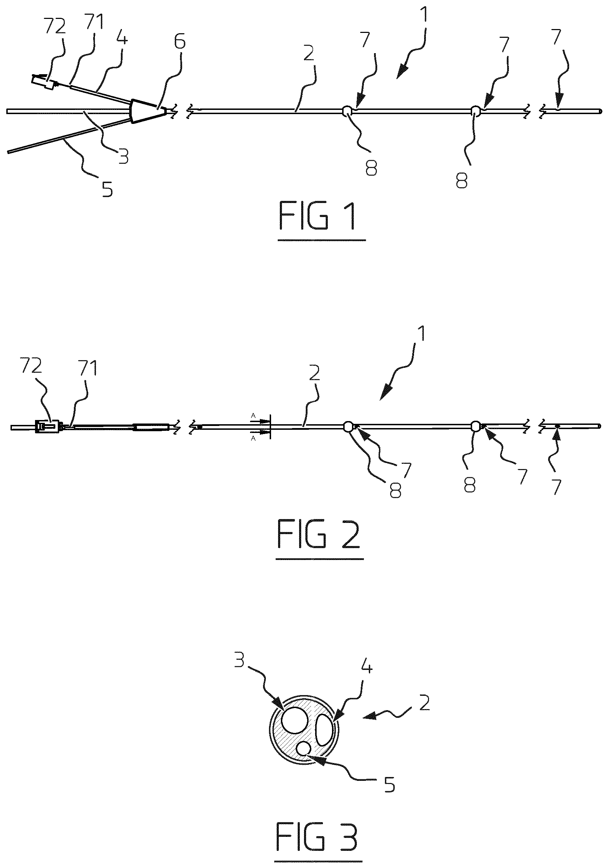

According to figures1 to 3 the esophageal pressure measurement catheter 1 comprises a flexible tube 2. The tube may is typically 95 - 125 cm long and approximately 4 mm in diameter. The tube is provided with an enteral feeding lumen 3, a sensor lumen 4 and a balloon inflation lumen 5, which exit the tube 2 through a trifurcation piece 6.

The enteral feeding lumen 3 is used to feed a patient with liquid food, administer medication and/or suction aspirate during the pressure measurement period, which may last for more than several days.

The pressure is measured by a plurality of solid state pressure sensors 7, which are distributed along the length of the tube 2 and which are in open communication with the esophagus of the patient though corresponding holes in the wall of the tube 2. The sensors 7 are connected by connection members 71 in the sensor lumen 4 to a sensor connector 72, for instance an RJ45 connector, at the proximal outer end of the sensor lumen 4.

Adjacent each pressure sensor 7 an inflatable medical balloon 8 is provided on the tube 2. The balloons 8 may for instance be made of urethane. The distance between the edge of the inflated balloon 8 and the centre of the pressure sensor is for instance approximately 4 mm, while the diameter of the inflated balloon is for instance approximately 12 to 15 mm. In that manner the balloon acts as a spacer, which prevents that the wall of the esophagus can touch or come near the pressure sensor 7.

In another preferred embodiment, a balloon 8 is provided at each side of each pressure sensor 7 at about 3 mm distance, whereby the spacer function that prevents that the wall of the esophagus can touch or come near the pressure sensor 7 is improved compared to the embodiment with one balloon 8 for each pressure sensor 7.

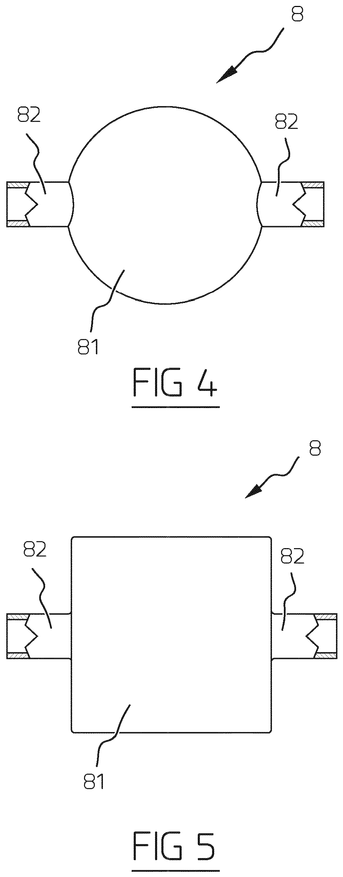

Figure 4 shows an example of a balloon 8, which comprises a spherical inflatable part 81 and two leg parts 82 at each end for mounting the balloon 8 on the tube 2. The leg parts are about 3 mm long in the axial direction.

Figure 5 shows another example of a balloon 8, which comprises a cylindrical inflatable part 81 and two leg parts 82 at each end for mounting the balloon 8 on the tube 2. The leg parts are about 3 mm long in the axial direction. The inflatable part 81 has a substantially rectangular longitudinal cross section, whereby the spacer function that prevents that the wall of the esophagus can touch or come near the pressure sensor 7 is improved compared to the embodiment of figure 4.

The invention has thus been described by means of preferred embodiments. It is to be understood, however, that this disclosure is merely illustrative.

## Claims

1. A catheter (1) comprising a tube (2) having a proximal end and a distal end, wherein the distal end of the tube (2) is arranged to be inserted into the esophagus of a patient, wherein the tube (2) is provided with at least one pressure sensor (7) for measuring a pressure in the esophagus region of a patient, wherein the tube (2) is provided with at least one inflatable balloon (8) adjacent to the at least one pressure sensor (7), wherein the catheter (1) is an esophageal pressure measurement catheter (1), wherein the tube (2) comprises a feeding lumen (3) for transporting liquid food, medication and/or gastric acid to and/or from the stomach of a patient, wherein the balloon (8), when inflated, has a diameter of between 7 mm and 18 mm , and wherein a distance between an edge of the balloon (8) and the centre of the pressure sensor (7) is less than 20 mm, such that the balloon (8), when inflated, acts as a spacer to keep the wall of the esophagus away from the pressure sensor (7)..

2. The esophageal pressure measurement catheter (1) in accordance with claim 1, wherein the tube (2) comprises a pressure sensor lumen (4) for accommodating a connection for the at least one pressure sensor (7).

3. The esophageal pressure measurement catheter (1) in accordance with claim 1 or 2, wherein the tube (2) comprises a balloon inflation lumen (5) for transporting a fluid, such as air, to the at least one inflatable balloon (8).

4. The esophageal pressure measurement catheter (1) in accordance with any of the previous claims, wherein the balloon (8), when inflated, has a diameter of between 10 mm and 15 mm.

5. The esophageal pressure measurement catheter (1) in accordance with any of the previous claims, wherein the distance between an edge of the balloon (8) and the centre of the pressure sensor (7) is less than 10 mm, preferably less than 7 mm, more preferably less than 5mm, most preferably less than 3 mm.

6. The esophageal pressure measurement catheter (1) in accordance with any of the previous claims, wherein the balloon (8) has a spherical shape, or the balloon (8) has a cylindrical shape with a substantially rectangular longitudinal cross section.

7. The esophageal pressure measurement catheter (1) in accordance with any of the previous claims, wherein the length of the tube (2) is at least 80 cm, preferably at least 95 cm.

8. The esophageal pressure measurement catheter (1) in accordance with any of the previous claims, wherein the diameter of the tube (2) is between 2 mm and 6 mm.

9. The esophageal pressure measurement catheter (1) in accordance with any of the previous claims, wherein said at least one pressure sensor (7) is a solid state pressure sensor.

10. The esophageal pressure measurement catheter (1) in accordance with any of the previous claims, wherein the balloon (8) is made of urethane, PET and/or nylon.

11. The esophageal pressure measurement catheter (1) in accordance with any of the previous claims, wherein the wall thickness of the balloon (8) is between 0.01 mm and 0.06 mm.

12. The esophageal pressure measurement catheter (1) in accordance with any of the previous claims, wherein the balloon (8) has a leg (82) on either side for fixating the balloon (8) on the tube (2), wherein the length of said leg (82) is less than 7 mm, preferably less than 5 mm, more preferably less than 3 mm in axial direction.

13. The esophageal pressure measurement catheter (1) in accordance with any of the previous claims, wherein the tube (2) is provided with two of said inflatable balloons (8) adjacent to and at each side of the at least one pressure sensor (7), wherein a distance between an edge of each of the balloons (8) and the centre of the pressure sensor (7) is less than 20 mm, preferably less than 10 mm, more preferably less than 7 mm, even more preferably less than 5mm, most preferably less than 3 mm.

14. The esophageal pressure measurement catheter (1) in accordance with any of the previous claims, wherein said tube (2) is provided with a plurality of said pressure sensors (7) distributed along a length of the tube (2), each pressure sensor (7) being provided with at least one inflatable balloon (8) adjacent to the at least one pressure sensor (7), wherein a distance between an edge of the balloon (8) and the centre of the pressure sensor (7) is less than 20 mm, preferably less than 10 mm, more preferably less than 7 mm, even more preferably less than 5mm, most preferably less than 3 mm.

## Patentansprüche

1. Ein Katheter (1), umfassend einen Schlauch (2) mit einem proximalen Ende und einem distalen Ende, wobei das distale Ende des Schlauchs (2) dafür eingerichtet ist, in die Speiseröhre eines Patienten eingeführt zu werden, wobei der Schlauch (2) mit mindestens einem Drucksensor (7) zum Messen eines Drucks im Speiseröhrenbereich eines Patienten versehen ist, wobei der Schlauch (2) mit mindestens einem aufblasbaren Ballon (8) benachbart zu dem mindestens einen Drucksensor (7) versehen ist, wobei der Katheter (1) ein Ösophagus-Druckmesskatheter (1) ist, wobei der Schlauch (2) ein Ernährungslumen (3) zum Transportieren von flüssiger Nahrung, Medikamenten und/oder Magensäure zum und/oder vom Magen eines Patienten umfasst, wobei der Ballon (8) im aufgeblasenen Zustand einen Durchmesser zwischen 7 mm und 18 mm aufweist, und wobei ein Abstand zwischen einem Rand des Ballons (8) und der Mitte des Drucksensors (7) weniger als 20 mm beträgt, so dass der Ballon (8) im aufgeblasenen Zustand als Abstandshalter wirkt, um die Wand der Speiseröhre vom Drucksensor (7) fernzuhalten.

2. Der Ösophagus-Druckmesskatheter (1) nach Anspruch 1, wobei der Schlauch (2) ein Drucksensorlumen (4) zur Aufnahme einer Verbindung für den mindestens einen Drucksensor (7) umfasst.

3. Der Ösophagus-Druckmesskatheter (1) nach Anspruch 1 oder 2, wobei der Schlauch (2) ein Ballon-Aufblaslumen (5) zum Transportieren eines Fluids, wie z. B. Luft, zu dem mindestens einen aufblasbaren Ballon (8) umfasst.

4. Der Ösophagus-Druckmesskatheter (1) nach einem der vorhergehenden Ansprüche, wobei der Ballon (8) im aufgeblasenen Zustand einen Durchmesser zwischen 10 mm und 15 mm aufweist.

5. Der Ösophagus-Druckmesskatheter (1) nach einem der vorhergehenden Ansprüche, wobei der Abstand zwischen einem Rand des Ballons (8) und der Mitte des Drucksensors (7) weniger als 10 mm, vorzugsweise weniger als 7 mm, weiter bevorzugt weniger als 5 mm, am meisten bevorzugt weniger als 3 mm beträgt.

6. Der Ösophagus-Druckmesskatheter (1) nach einem der vorhergehenden Ansprüche, wobei der Ballon (8) eine kugelförmige Form aufweist, oder der Ballon (8) eine zylindrische Form mit einem im Wesentlichen rechteckigen Längsschnitt aufweist.

7. Der Ösophagus-Druckmesskatheter (1) nach einem der vorhergehenden Ansprüche, wobei die Länge des Schlauchs (2) mindestens 80 cm, vorzugsweise mindestens 95 cm beträgt.

8. Der Ösophagus-Druckmesskatheter (1) nach einem der vorhergehenden Ansprüche, wobei der Durchmesser des Schlauchs (2) zwischen 2 mm und 6 mm beträgt.

9. Der Ösophagus-Druckmesskatheter (1) nach einem der vorhergehenden Ansprüche, wobei der mindestens eine Drucksensor (7) ein Festkörper-Drucksensor ist.

10. Der Ösophagus-Druckmesskatheter (1) nach einem der vorhergehenden Ansprüche, wobei der Ballon (8) aus Urethan, PET und/oder Nylon hergestellt ist.

11. Der Ösophagus-Druckmesskatheter (1) nach einem der vorhergehenden Ansprüche, wobei die Wanddicke des Ballons (8) zwischen 0,01 mm und 0,06 mm beträgt.

12. Der Ösophagus-Druckmesskatheter (1) nach einem der vorhergehenden Ansprüche, wobei der Ballon (8) auf jeder Seite einen Schenkel (82) zum Befestigen des Ballons (8) am Schlauch (2) aufweist, wobei die Länge des Schenkels (82) in axialer Richtung weniger als 7 mm, vorzugsweise weniger als 5 mm, weiter bevorzugt weniger als 3 mm beträgt.

13. Der Ösophagus-Druckmesskatheter (1) nach einem der vorhergehenden Ansprüche, wobei der Schlauch (2) mit zwei der aufblasbaren Ballons (8) benachbart zu und auf jeder Seite von dem mindestens einen Drucksensor (7) versehen ist, wobei ein Abstand zwischen einem Rand jedes der Ballons (8) und der Mitte des Drucksensors (7) weniger als 20 mm, vorzugsweise weniger als 10 mm, weiter bevorzugt weniger als 7 mm, noch weiter bevorzugt weniger als 5 mm, am meisten bevorzugt weniger als 3 mm beträgt.

14. Der Ösophagus-Druckmesskatheter (1) nach einem der vorhergehenden Ansprüche, wobei der Schlauch (2) mit einer Vielzahl der Drucksensoren (7) versehen ist, die entlang einer Länge des Schlauchs (2) verteilt sind, wobei jeder Drucksensor (7) mit mindestens einem aufblasbaren Ballon (8) benachbart zu dem mindestens einen Drucksensor (7) versehen ist, wobei ein Abstand zwischen einem Rand des Ballons (8) und der Mitte des Drucksensors (7) weniger als 20 mm, vorzugsweise weniger als 10 mm, weiter bevorzugt weniger als 7 mm, noch weiter bevorzugt weniger als 5 mm, am meisten bevorzugt weniger als 3 mm beträgt.

## Revendications

1. Un cathéter (1) comprenant un tube (2) ayant une extrémité proximale et une extrémité distale, dans lequel l'extrémité distale du tube (2) est agencée pour être insérée dans l'œsophage d'un patient, dans lequel le tube (2) est pourvu d'au moins un capteur de pression (7) pour mesurer une pression dans la région de l'œsophage d'un patient, dans lequel le tube (2) est pourvu d'au moins un ballonnet gonflable (8) adjacent à l'au moins un capteur de pression (7), dans lequel le cathéter (1) est un cathéter de mesure de pression œsophagienne (1), dans lequel le tube (2) comprend une lumière d'alimentation (3) pour transporter un aliment liquide, un médicament et/ou de l'acide gastrique vers et/ou depuis l'estomac d'un patient, dans lequel le ballonnet (8), lorsqu'il est gonflé, a un diamètre compris entre 7 mm et 18 mm, et dans lequel une distance entre un bord du ballonnet (8) et le centre du capteur de pression (7) est inférieure à 20 mm, de telle sorte que le ballonnet (8), lorsqu'il est gonflé, agit comme un écarteur pour maintenir la paroi de l'œsophage à l'écart du capteur de pression (7).

2. Le cathéter de mesure de pression œsophagienne (1) selon la revendication 1, dans lequel le tube (2) comprend une lumière de capteur de pression (4) pour loger une connexion pour l'au moins un capteur de pression (7).

3. Le cathéter de mesure de pression œsophagienne (1) selon la revendication 1 ou 2, dans lequel le tube (2) comprend une lumière de gonflage de ballonnet (5) pour transporter un fluide, tel que de l'air, vers l'au moins un ballonnet gonflable (8).

4. Le cathéter de mesure de pression œsophagienne (1) selon l'une quelconque des revendications précédentes, dans lequel le ballonnet (8), lorsqu'il est gonflé, a un diamètre compris entre 10 mm et 15 mm.

5. Le cathéter de mesure de pression œsophagienne (1) selon l'une quelconque des revendications précédentes, dans lequel la distance entre un bord du ballonnet (8) et le centre du capteur de pression (7) est inférieure à 10 mm, de préférence inférieure à 7 mm, plus préférablement inférieure à 5 mm, le plus préférablement inférieure à 3 mm.

6. Le cathéter de mesure de pression œsophagienne (1) selon l'une quelconque des revendications précédentes, dans lequel le ballonnet (8) a une forme sphérique, ou le ballonnet (8) a une forme cylindrique avec une section transversale longitudinale sensiblement rectangulaire.

7. Le cathéter de mesure de pression œsophagienne (1) selon l'une quelconque des revendications précédentes, dans lequel la longueur du tube (2) est d'au moins 80 cm, de préférence d'au moins 95 cm.

8. Le cathéter de mesure de pression œsophagienne (1) selon l'une quelconque des revendications précédentes, dans lequel le diamètre du tube (2) est compris entre 2 mm et 6 mm.

9. Le cathéter de mesure de pression œsophagienne (1) selon l'une quelconque des revendications précédentes, dans lequel ledit au moins un capteur de pression (7) est un capteur de pression à semi-conducteurs.

10. Le cathéter de mesure de pression œsophagienne (1) selon l'une quelconque des revendications précédentes, dans lequel le ballonnet (8) est fait d'uréthane, de PET et/ou de nylon.

11. Le cathéter de mesure de pression œsophagienne (1) selon l'une quelconque des revendications précédentes, dans lequel l'épaisseur de paroi du ballonnet (8) est comprise entre 0,01 mm et 0,06 mm.

12. Le cathéter de mesure de pression œsophagienne (1) selon l'une quelconque des revendications précédentes, dans lequel le ballonnet (8) a une patte (82) de chaque côté pour fixer le ballonnet (8) sur le tube (2), dans lequel la longueur de ladite patte (82) est inférieure à 7 **mm,** de préférence inférieure à 5 **mm,** plus préférablement inférieure à 3 mm en direction axiale.

13. Le cathéter de mesure de pression œsophagienne (1) selon l'une quelconque des revendications précédentes, dans lequel le tube (2) est pourvu de deux desdits ballonnets gonflables (8) adjacents à et de chaque côté de l'au moins un capteur de pression (7), dans lequel une distance entre un bord de chacun des ballonnets (8) et le centre du capteur de pression (7) est inférieure à 20 mm, de préférence inférieure à 10 **mm,** plus préférablement inférieure à 7 mm, encore plus préférablement inférieure à 5 mm, le plus préférablement inférieure à 3 mm.

14. Le cathéter de mesure de pression œsophagienne (1) selon l'une quelconque des revendications précédentes, dans lequel ledit tube (2) est pourvu d'une pluralité desdits capteurs de pression (7) répartis le long d'une longueur du tube (2), chaque capteur de pression (7) étant pourvu d'au moins un ballonnet gonflable (8) adjacent à l'au moins un capteur de pression (7), dans lequel une distance entre un bord du ballonnet (8) et le centre du capteur de pression (7) est inférieure à 20 mm, de préférence inférieure à 10 **mm,** plus préférablement inférieure à 7 mm, encore plus préférablement inférieure à 5 mm, le plus préférablement inférieure à 3 mm.
